(19)

Europäisches
Patentamt
European
Patent Office
Office européen
des brevets

(11) **EP 3 457 353 B1**

(12) **EUROPEAN PATENT SPECIFICATION**

(45) Date of publication and mention
of the grant of the patent:
**25.11.2020 Bulletin 2020/48**

(51) Int Cl.:
***G06T 5/00*** *(2006.01)*      ***A61B 6/00*** *(2006.01)*

(21) Application number: **17191582.0**

(22) Date of filing: **18.09.2017**

(54) **METHOD AND SYSTEM FOR OBTAINING A TRUE SHAPE OF OBJECTS IN A MEDICAL IMAGE**

VERFAHREN UND SYSTEM ZUM ERHALTEN EINER ECHTEN FORM VON OBJEKTEN IN EINEM
MEDIZINISCHEN BILD

PROCÉDÉ ET SYSTÈME POUR L'OBTENTION DE FORME RÉELLE D'OBJETS DANS UNE IMAGE
MÉDICALE

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.03.2019 Bulletin 2019/12**

(73) Proprietor: **Siemens Healthcare GmbH
91052 Erlangen (DE)**

(72) Inventors:
 • **Tripathi, Gaurav
  226002 Lucknow, Uttar Pradesh (IN)**
 • **Betharia, Mayur
  560083 Bangalore, Karnataka (IN)**

(74) Representative: **Patentanwälte Bals & Vogel
Sendlinger Strasse 42A
80331 München (DE)**

(56) References cited:
 **US-A1- 2008 063 304**     **US-B1- 6 289 235**

 • **DOBBINS J T ET AL: "Digital x-ray
  tomosynthesis: current state of the art and
  clinical potential", PHYSICS IN MEDICINE AND
  BIOLOGY, INSTITUTE OF PHYSICS PUBLISHING,
  BRISTOL GB, vol. 48, no. 19, 7 October 2003
  (2003-10-07), pages R65-R106, XP002277335,
  ISSN: 0031-9155, DOI:
  10.1088/0031-9155/48/19/R01**
 • **KOLITSI Z ET AL: "A MULTIPLE PROJECTION
  METHOD FOR DIGITAL TOMOSYNTHESIS",
  MEDICAL PHYSICS, AIP, MELVILLE, NY, US, vol.
  19, no. 4, July 1992 (1992-07), pages 1045-1050,
  XP000309453, ISSN: 0094-2405, DOI:
  10.1118/1.596822**
 • **National Electrical Manufacturers Association:
  "DICOM Standard, part PS3.3 (chapter C.8.19.6.9,
  X-Ray Projection Pixel Calibration Macro)", ,
  2004, XP055433169, Retrieved from the Internet:
  URL:http://dicom.nema.org/medical/dicom/cu
  rrent/output/pdf/part03.pdf [retrieved on
  2017-12-07]**

---

**Description**

The present invention relates to a method and hardware and a software system of obtaining a true shape of an object in a medical image. Said method includes selecting a plurality of pixels representing an object in said medical image and determining magnification of each pixel of said object in said medical image.

Pixel spacing represents the physical distance between two adjacent pixels. In order to measure an object in a medical image, such as an X-ray image, the pixel spacing has to be accurately determined. Image calibration techniques well known in the prior art determine the pixel spacing by assuming the patient to be positioned in the two-dimensional plane perpendicular to the central axis of the X-ray beam. Determination of pixel spacing in an X-ray image may be performed by several techniques that may be well known to a person skilled in the art. For example, a geometric calibration may be performed where magnification is determined based on the position of X-ray tube, X-ray detector, object being examined, and image iso-center. This approach determines the geometric beam path from X-ray source to X-ray detector and calculates the magnification of said object interfering with the beam. In an alternative method, pixel spacing is determined by dividing a known length of said object with the pixels representing said object. The abovementioned calibration techniques assume the object magnification within the X-ray image to be uniform. However, if said object plane is not perpendicular to said axis of the X-ray beam, the magnification across said X-ray image may not be uniform. Therefore, due to variable magnification across the image, the measurements obtained of said object in said X-ray image are inaccurate. Furthermore, variable magnification may also render said objects in said X-ray image to be deformed. This may also cause error in registering the X-ray image with other modality in hybrid procedures.

The object of the invention is therefore to correct the magnification across a medical image so as to accurately determine the size and shape of the imaged objects.

J. T. Dobbins III and D. J. Godfrey, in "Digital x-ray tomosynthesis: current state of the art and clinical potential", Phys. Med. Biol. 48, 2003, reviews digital x-ray tomosynthesis methods, referring in particular to the need for image rectification and magnification correction and citing Z. Kolitsi et al., "A multiple projection method for digital tomosynthesis", Med. Phys. 19(4), 1992, disclosing a method for digital X-ray tomosynthesis for warping specific captures corresponding to different detector angles.

DICOM Standard, part PS3.3, version 2014b, National Manufacturers Association, states formats for calibration of X-ray related images and includes diagrams indicating the calculations required for achieving object size measurements from angulated captures.

US 2008/063304 A1 describes the use of calibrating markers, and more specifically of spherical metal markers, to correct the magnification of X-ray captures.

Based on the aforementioned methods, the invention achieves said object by determining an actual size of said object in said medical image and selectively reshaping said object in said medical image to obtain a true shape of said object.

The present invention describes a method of obtaining a true shape of an object in a medical image according to appended claim 1. The method comprises receiving X-ray stand geometry parameter values, an $Angle_{point}$, and an $Angle_{stand}$ values. Said X-ray stand geometry parameter values, an $Angle_{point}$, and an $Angle_{stand}$ values may be received, for example, from an imaging unit during acquisition of medical image. Alternatively, if said medical image is an image which is obtained from a database, said stand parameters and angle values may be obtained from DICOM data. According to an embodiment of the invention, the method further comprises selecting a plurality of pixels representing an object in said medical image. Said medical image is an X-ray image. Said X-ray image may be a conventional radiographic image or a live fluoroscopic X-ray image.

According to an embodiment of the invention, in selecting a plurality of pixels representing said object on said medical image, a plurality of pixels representing said object is identified and from said plurality of identified pixels, a sequence of connecting pixels which represent a contour of said object is selected. Said plurality of pixels may be chosen by a user by drawing a contour on said medical image by dragging a cursor around said desired object. On drawing said contour, a plurality of pixels in said desired area on said object is selected. Alternatively, said plurality of pixels representing said object may be identified automatically by use of one or more landmarks for object identification. Said contour of said object may therefore be selected automatically based on, for example, shape based models or appearance based models for object segmentation. Selection of a plurality of pixels in said X-ray image enables identification a region of interest in said X-ray image.

According to another embodiment of the invention, said method further comprises determining an actual size of said object on said medical image. Said actual size of said object is determined by calculating pixel spacing for each pixel representing said contour. Pixel spacing is a physical distance between the centers of two two-dimensional pixels. Calculating pixel spacing for each pixel along said contour of said object enables determination of actual size of said object accurately. According to the state of the art, size of an object in an X-ray image is determined using fixed pixel spacing between two pixels. The determined pixel spacing value between two adjacent pixels is assumed to be uniform across said X-ray image. However, if magnification factor across said pixels is different, use of fixed pixel spacing to determine actual size of said object may provide erroneous results. Therefore, calculating pixel spacing values for each pixel along said contour of said object enables determination of actual size of said object irrespective of change in magnification of pixels.

The object of the invention is also achieved by a system for obtaining a true shape of an object in a medical image according to appended claim 4.

According to an embodiment of the invention, said system comprises a processing unit and an image database coupled to said processing unit. Said image database includes one or more medical images that may have been acquired from a medical imaging unit or, for example, obtained from an external source. Said system further comprises a memory coupled to said processing unit. Said memory comprises a magnification correction module. Said magnification correction module is configured to receive X-ray stand geometry parameter values, an Anglepoint, and an Anglestand values. Said X-ray stand geometry parameter values, an $Angle_{point}$, and an $Angle_{stand}$ values may be received, for example, from an imaging unit during acquisition of medical image. Alternatively, if said medical image is an image which is obtained from a database, said stand parameters and angle values may be obtained from DICOM data.

According to an embodiment of the invention, said magnification correction module is further configured to select a plurality of pixels representing an object in said medical image. Said medical image is an X-ray image. Said X-ray image may be a conventional radiographic image or a live fluoroscopic X-ray image.

According to an embodiment of the invention, in selecting a plurality of pixels representing said object on said medical image, said magnification correction module is configured to identify a plurality of pixels representing said object and select, from said plurality of identified pixels, a sequence of connecting pixels which represent a contour of said object. Said plurality of pixels may be chosen by a user by drawing a contour on said medical image by dragging a cursor around said desired object. On drawing said contour, a plurality of pixels in said desired area on said object is selected. Alternatively, said plurality of pixels representing said object may be identified automatically by use of one or more landmarks for object identification. Said contour of said object may therefore be selected automatically based on, for example, shape based models or appearance based models for object segmentation. Selection of a plurality of pixels in said X-ray image enables identification a region of interest in said X-ray image.

According to another embodiment of the invention, said magnification correction module is further configured to determine an actual size of said object on said medical image. Said actual size of said object is determined by calculating pixel spacing for each pixel representing said contour. Pixel spacing is a physical distance between the centers of two two-dimensional pixels. Calculating pixel spacing for each pixel along said contour of said object enables determination of actual size of said object accurately. According to the state of the art, size of an object in an X-ray image is determined using fixed pixel spacing between two pixels. The determined pixel spacing value between two adjacent pixels is assumed to be uniform across said X-ray image. However, if magnification factor across said pixels is different, use of fixed pixel spacing to determine actual size of said object may provide erroneous results. Therefore, calculating pixel spacing values for each pixel along said contour of said object enables determination of actual size of said object irrespective of change in magnification of pixels.

Also disclosed herein is a computer program product comprising a non-transitory computer-readable storage medium having machine-readable instructions stored therein, that when executed by a server, cause the server to perform the method steps for obtaining a true shape of objects in a medical image according to appended claim 7.

The present invention is further described hereinafter with reference to illustrated embodiments shown in the accompanying drawings, in which:

FIG 1    illustrates a block diagram of a data processing system in which an embodiment for obtaining a true shape of an object in an X-ray image can be implemented.

FIG 2    illustrates a flowchart of an embodiment of a method of obtaining a true shape of an object in an X-ray image.

FIG 3    illustrates a flowchart of an embodiment of a method of selectively reshaping said object in said X-ray image.

FIG 4A   illustrates an embodiment of an X-ray image in which an imaged object is deformed due to variation in magnification across said X-ray image.

FIG 4B   illustrates a table indicating the percentage error in the values of measurement of objects in said X-ray image.

FIG 4C   illustrates an embodiment of a medical imaging system.

FIG 5    illustrates an embodiment of a graph providing a comparison of object measurements performed on said X-ray image using the proposed method over techniques existing in the prior art.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

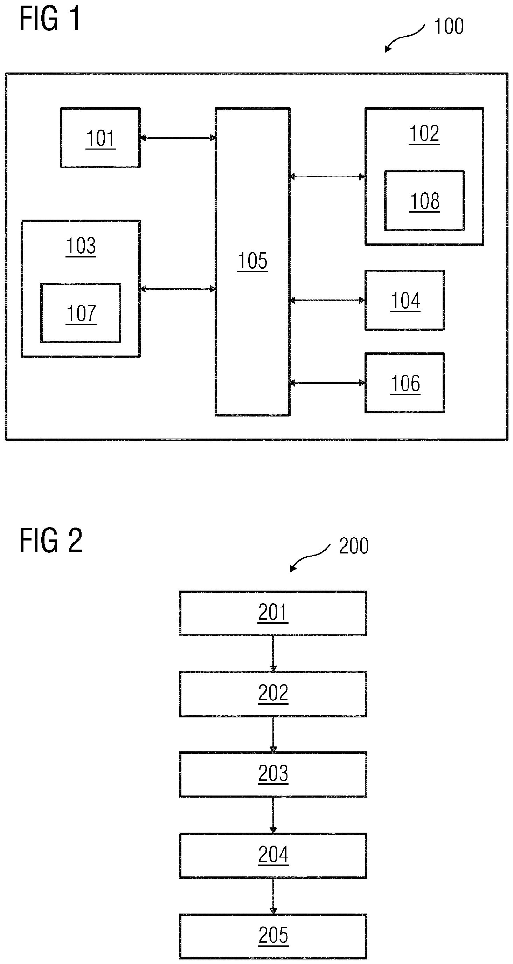

FIG 1 is a block diagram of a data processing system 100 in which an embodiment can be implemented, for example, as a system 100 for obtaining true shape of an object, configured to perform the processes as described therein. In FIG 1, said data processing system 100 comprises a processor 101, a memory 102, a storage unit 103, an input unit 104, an output unit 106 and a bus 105.

Said processor 101, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. Said processor 101 may also include embedded controllers, such as generic or programmable logic devices or arrays, application specific integrated circuits, single-chip computers, and the like.

Said memory 102 may be volatile memory and non-volatile memory. Said memory 102 may be coupled for communication with said processor 101. Said processor 101 may execute instructions and/or code stored in said memory 102. A variety of computer-readable storage media may be stored in and accessed from said memory 102. Said memory 102 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, said memory 102 includes a magnification correction module 108 stored in the form of machine-readable instructions on any of said above-mentioned storage media and may be in communication to and executed by processor 101. When executed by said processor 101, said magnification correction module 108 causes said processor 101 to perform correction of magnification of a plurality of pixels in an X-ray image. Method steps executed by said processor 101 to achieve the abovementioned functionality are elaborated upon in detail in FIG 2, 3, 4A, 4B and 4C.

Said storage unit 103 may be a non-transitory storage medium which stores an image database 107. Said image database 107 is a repository of medical images related to one or more patients that is maintained by a healthcare service provider. Said input unit 104 may include input means such as keypad, touch-sensitive display, camera (such as a camera receiving gesture-based inputs), etc. capable of receiving input signal such as a medical image. Said bus 105 acts as interconnect between said processor 101, said memory 102, said storage unit 103, said input unit 104 and said output unit 106.

Those of ordinary skilled in the art will appreciate that said hardware depicted in FIG 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 100 in accordance with an embodiment of the present disclosure includes an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows™, a product of Microsoft Corporation located in Redmond, Washington may be employed if suitably modified. Said operating system is modified or created in accordance with the present disclosure as described.

Disclosed embodiments provide systems and methods for providing a true shape of objects in medical images. In particular, said systems and methods may perform magnification correction of a plurality of pixels in a medical image.

FIG 2 illustrates a flowchart of an embodiment of a method 200 of obtaining a true shape of an object in a medical image. Said medical image is an X-ray image.

At step 201, an X-ray image is received from said image database 107 present in said storage unit 103. Said X-ray image is acquired from an imaging unit, such as an X-ray unit, and is stored in said image database 107. Said X-ray image may be acquired from an X-ray unit in which X-ray source and detector are positioned at an angle. Therefore, said X-ray source and said detector are not placed perpendicular to the object plane. Therefore, the magnification across said acquired X-ray image is varied because the object plane is not perpendicular to the axis of the X-ray beam. Thus, said acquired X-ray image may not depict a true shape of said imaged object.

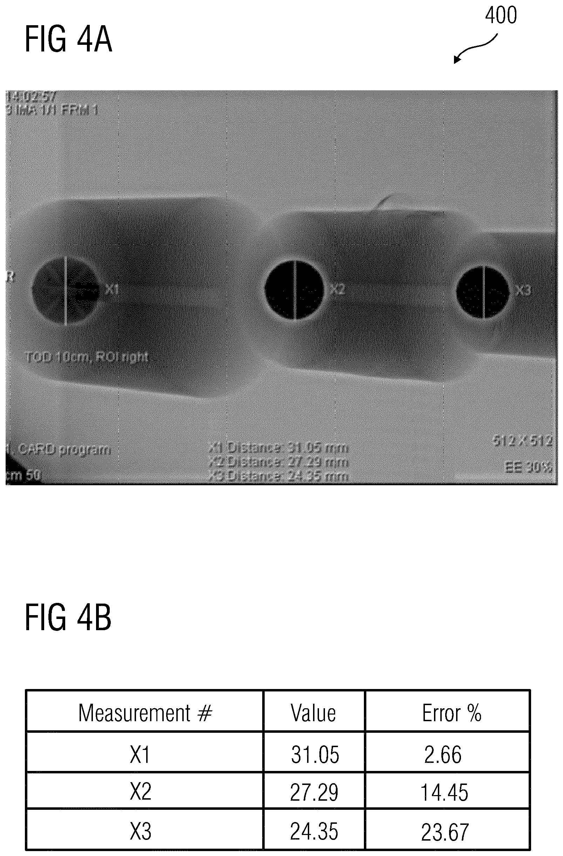

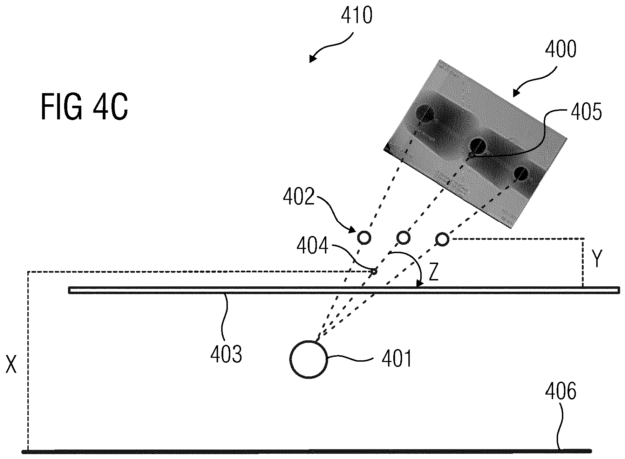

FIG 4A illustrates an embodiment of an acquired X-ray image 400 in which an imaged object is deformed due to variation in magnification across said X-ray image. Said X-ray image 400 depicts three spheres, X1, X2 and X3, each sphere having an original diameter of 31.90 millimeter. However, the size of said spheres in said acquired X-ray image 400 appears different from each other. In said X-ray image 400, X1 appears bigger than X2 and X3. Furthermore, X1 appears to be deformed in comparison to X2 and X3. Due to varied magnification, contour of X1 does not appear to be perfectly circular. FIG 4C illustrates an embodiment of a medical imaging system 410. Said medical imaging system 410 is an X-ray imaging system, in the present embodiment. Said X-ray imaging system 410 includes an X-ray source 401, one or more objects 402, an X-ray detector on which an image 400 of said object 402 is captured, a table 403 on which an object or patient to be imaged is placed and a floor 406. 404 is the iso-center and 405 is the center of the detector. Variable X depicts the height of the iso-center from the floor 406 and variable Y depicts the height of the object 402 from the table 403. The angle formed by the line joining the center of the X-ray source 401 to the center of the detector 405 with the horizontal table is depicted as Z and is known as the Angle$_{stand}$.

The percentage of error in the diameter values of X1, X2 and X3 is indicated in a table illustrated in FIG 4B. In said table, 'Value' indicates a measured diameter value of said respective sphere in said X-ray image 400. The column 'Error%' indicates the percentage of deviation of said measured diameter value of each sphere from said actual/original diameter of said sphere.

At step 202 of said method 200, a plurality of pixels that represent an object on said X-ray image is chosen. Said plurality of pixels is alternatively referred to as a contour. Said plurality of pixels is chosen automatically by using any organ segmentation method that may be known to a person skilled in the art. Alternatively, said contour may be defined by a user by dragging a cursor along said desired object. Dragging said cursor along said desired object enables selection of a plurality of pixels representing said contour of said object in said X-ray image.

At step 203, magnification factor of each pixel in said X-ray image is determined. Magnification factor of a pixel in an X-ray image is determined using the below mathematical equation:

$$M = K/\cos (Angle_{point})/\sin (Angle_{stand} + Angle_{point})$$

Where:

M: Magnification factor;

K: Stand geometry parameters which remain constant for X-ray source 401, X-ray detector, and object position 402 for a single X-ray frame;

Angle$_{point}$: An angle which a line joining X-ray source 401 to measured point (on said X-ray image) makes with a line joining X-ray source to the center of the X-ray image;

Angle$_{stand}$: An angle Z which a line connecting said X-ray source 401 and an X-ray detector makes with a horizontal axis - said angle remains constant in a single X-ray image.

Said stand geometry parameters, Angle$_{point}$, and Angle$_{stand}$ are obtained from said imaging unit during the acquisition of said medical image. Alternatively, if said medical image is an image which is obtained from a database, said stand parameters and angle values may be obtained from DICOM data.

Magnification factor in an X-ray image may vary across pixels. Therefore, bringing uniformity in said magnification factor across said X-ray image enables accurate determination of size of said object in said X-ray image. At step 204 of said method 200, pixel spacing of each pixel representing said contour is determined. Pixel spacing is the physical distance between the centers of two adjacent pixels. Instead of using fixed pixel spacing, determination of pixel spacing between each set of adjacent pixels enables determination of actual size of said object in said X-ray image. Pixel spacing between adjacent pixels may be determined using the following equation:

$$P_{a(i,j)} = P_{o(i,j)} + M_{(i,j)}$$

Where:

$P_{a(i,j)}$ : Actual pixel spacing between two adjacent pixels (i,j) of said X-ray image;

$P_{o(i,j)}$: X-ray detector pixel spacing between two adjacent pixels (i,j) of said X-ray image stored during X-ray acquisition

$M_{(i,j)}$: Magnification factor of said object calculated between two adjacent pixels (i,j) taking that point as region of interest.

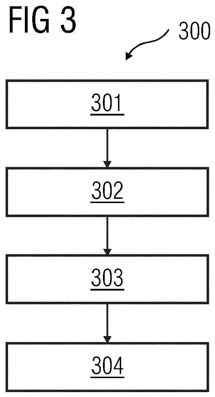

Once said actual size of said object is determined, at step 250, said object is selectively reshaped to obtain a true shape of said object. The method steps involved in selectively reshaping said object in said X-ray image are explained in detail in FIG 3.

FIG 3 illustrates a flowchart of an embodiment of a method 300 of selectively reshaping an object in an X-ray image. At step 301, a region of interest on said X-ray image, which may be deformed, is chosen for reshaping. Said region of interest may be selected by said user during live acquisition of said X-ray image. The region of interest on the patient body may be chosen using X-ray stand position, beam iso-center and table-object distance Y as inputs. Said region of interest may also be chosen by said user during post processing of said X-ray image. On selection of said region of interest, at step 302, a reference pixel from said region of interest is chosen such that said reference pixel is the center of said region of interest. Said reference pixel may be any pixel within said region of interest. The magnification factor of said reference pixel is determined, based on which reshaping of said region of interest is performed. At step 303, the magnification of a plurality of pixels around said reference pixel is adjusted according to the magnification of said reference pixel. Therefore, said plurality of pixels around said reference pixel may be stretched or shrunk, thereby generating a sequence of uniformly magnified pixels across said X-ray image. At step 304, a rendering application renders the true shape of said object in said X-ray image using said uniformly magnified pixels. Said uniformly magnified pixels enable reshaping of said deformed object in said X-ray image. Therefore, a true shape of said object in said X-ray image is achieved. In an embodiment, the true shape of the object in said X-ray image is displayed on the output unit 106. Said output unit 106 may be, for example, a display unit. Advantageously, the invention achieves determination of true shape of an object in a medical image during real-time acquisition of said medical image. For example, in a real-time fluoroscopic imaging, the true shape of the object being imaged is determined during the interventional procedure. The obtained true shape of the object is displayed on said display unit 106 for further analysis by a physician. Therefore, the physician receives accurate imaging information, thereby avoiding the delay in post processing of the medical image. Thus, each time an image is acquired, the true shape of the object being imaged is determined and displayed to the physician in real-time. In an embodiment, the image displayed on said display unit 106 is refreshed with each acquisition.

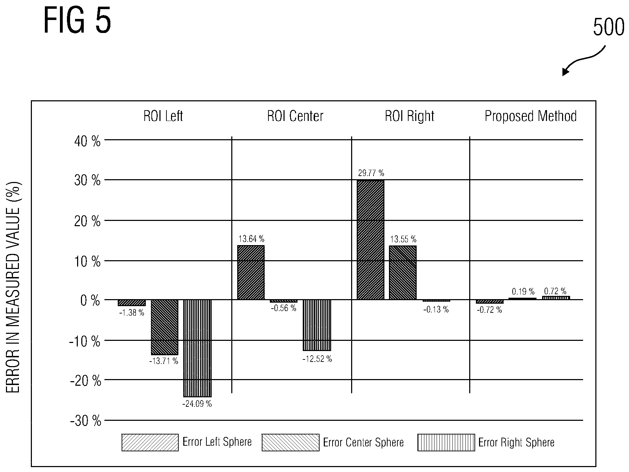

FIG 5 illustrates a graph 500 which provides a comparison of object measurements performed on said X-ray image using the proposed method over techniques existing in the prior art. In techniques existing in the prior art, a constant calibration factor is used across said X-ray image. Reference points from each sphere, X1, X2 and X3 are used for calibrating said X-ray image. When a magnification factor and pixel spacing value of a reference point from sphere X1 is used as a constant calibration factor for calibrating said X-ray image, the percentage of error in spheres X1, X2 and X3 is -1.38%, -13.71% and -24.09% respectively. When a magnification factor and pixel spacing value of reference point from X2 is used as a constant calibration factor for calibrating said X-ray image, the percentage of error in spheres X1, X2 and X3 is 13.64%, - 0.56% and -12.52% respectively. When a magnification factor and pixel spacing value of reference point from X3 is used as a constant calibration factor for calibrating said X-ray image, the percentage of error in spheres X1, X2 and X3 is 29.77%, 13.55% and -0.13% respectively. However, when said X-ray image is calibrated according to the proposed method in the invention, pixel spacing between each set of adjacent pixels making up said contour of said object, is determined in order to identify said actual size of said object. Furthermore, the magnification factor of said reference pixel in said region of interest is used for adjusting the magnification factor of a plurality of pixels around said reference pixel. Therefore, said plurality of pixels in said X-ray image are uniformly magnified, thereby providing an accurately calibrated X-ray image. When said proposed method is used for calibrating said X-ray image, the percentage of error in said determined true shape of said object is negligible. When a pixel from X1 is used as a reference pixel for calibrating said X-ray image, the percentage of error is -0.72%, 0.19% and 0.72% respectively for X1, X2 and X3. Therefore, said proposed method enables accurate determination of actual shape of an object in an X-ray image.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, it extends to all functionally equivalent structures, methods and uses, as long as they are within the scope of the invention, defined by the appended claims.

List of reference numbers

[0039]

100 System for obtaining true shape of an object
101 Processor
102 Memory
103 Storage unit
104 Input unit
105 Bus
106 Output unit
107 Image database
108 Magnification correction module
200 Method of obtaining true shape of object

300 Method of selectively reshaping an object on medical image
400 X-ray image
401 X-ray source
402 Objects
403 Table
404 Iso-center
405 Center of an X-ray detector
406 Floor
410 Medical imaging system
500 Graph comparing object measurements performed on an X-ray image using the proposed method over techniques existing in the prior art.

**Claims**

1. A method (200) of obtaining a true shape of an object in a medical X-ray image (400), said method (200) comprising:
   receiving a medical X-ray image (400) of the object;

   a. receiving X-ray stand geometry parameter values, an $Angle_{point}$, and an $Angle_{stand}$ values; wherein said $Angle_{point}$ is an angle which a line joining an X-ray source (401) to a measured point on said medical X-ray image (400) makes with a line joining X-ray source (401) to the center of said medical X-ray image (400); and wherein $Angle_{stand}$ is an angle Z which a line connecting said X-ray source (401) and an X-ray detector makes with a horizontal axis, said angle remaining constant in a single medical X-ray image (400);
   b. selecting a plurality of pixels representing said object in said medical X-ray image (400);
   c. determining magnification of each pixel in said medical X-ray image (400) using said stand geometry parameters, $Angle_{point}$, and an $Angle_{stand}$ values;
   **characterized by**:
   d. selectively reshaping said object in said medical X-ray image (400) to obtain a true shape of said object using said determined magnification, wherein reshaping said object in said medical X-ray image (400) further comprises:
   e. selecting a plurality of pixels representing a region of interest on said medical X-ray image (400);
   f. selecting a reference pixel in said region of interest; and
   g. adjusting said magnification of said plurality of pixels present in the surrounding of said reference pixel with respect to the magnification of said reference pixel.

2. The method (200) according to claim 1, wherein in selecting a plurality of pixels representing said object said method comprises:

   a. identifying a plurality of pixels representing said object in said medical X-ray image (400); and
   b. selecting from said plurality of pixels a sequence of connecting pixels which represent a contour of said object.

3. The method (200) according to claim 2, further comprising determining an actual size of said object by calculating pixel spacing for each pixel representing said contour, wherein pixel spacing is the physical distance between the centers of two adjacent pixels, and
   wherein pixel spacing between adjacent pixels is determined using the magnification of the pixels.

4. A system (100) for obtaining a true shape of an object in a medical X-ray image (400), said system (100) comprising:

   a. a processing unit (101);
   b. an image database (107) coupled to said processing unit (101);
   c. a memory (102) coupled to said processing unit (101), said memory (102) comprising a magnification correction module (108) configured to:
   receive a medical X-ray image (400) of the object;

   i. receive X-ray stand geometry parameter values, an $Angle_{point}$, and an $Angle_{stand}$ values; wherein said $Angle_{point}$ is an angle which a line joining an X-ray source (401) to a measured point on said medical X-ray image (400) makes with a line joining X-ray source (401) to the center of said medical X-ray image (400); and wherein $Angle_{stand}$ is an angle Z which a line connecting said X-ray source (401) and an X-ray detector makes with a horizontal axis, said angle remaining constant in a single medical X-ray image (400);

ii. select a plurality of pixels representing said object in said medical X-ray image (400);

iii. determine magnification of each pixel in said medical X-ray image (400) using said stand geometry parameters, Angle$_{point}$, and an Angle$_{stand}$ values;

**characterized in that** said magnification correction module (108) is further configured to:

selectively reshape said object in said medical X-ray image (400) to obtain a true shape of said object using said determined magnification,

wherein in selectively reshaping said object in said medical X-ray image (400), said magnification correction module (108) is configured to:

a. select a plurality of pixels representing a region of interest on said X-ray medical image (400);

b. select a reference pixel in said region of interest; and

c. adjust said magnification of said plurality of pixels present in the surrounding of said reference pixel with respect to the magnification of said reference pixel.

5. The system (100) according to claim 4, wherein in selecting a plurality of pixels representing said object said magnification correction module (108) is configured to:

a. identify a plurality of pixels representing said object in said medical X-ray image (400); and

b. select from said plurality of pixels a sequence of connecting pixels which represent an contour of said object.

6. The system (100) according to claim 5, wherein said magnification correction module is further configured to determine an actual size of said object by calculating pixel spacing for each pixel representing said contour, wherein pixel spacing is the physical distance between the centers of two adjacent pixels, and wherein pixel spacing between adjacent pixels is determined using the magnification of the pixels.

7. A non-transitory computer-readable storage medium having machine-readable instructions stored therein which, when executed by a server, cause the server to perform the method steps comprising: receiving a medical X-ray image (400) of an object;

a. receiving X-ray stand geometry parameter values, an Angle$_{point}$, and an Angle$_{stand}$ values; wherein said Angle$_{point}$ is an angle which a line joining an X-ray source (401) to a measured point on a medical X-ray image (400) makes with a line joining X-ray source (401) to the center of said medical X-ray image (400); and wherein Angle$_{stand}$ is an angle Z which a line connecting said X-ray source (401) and an X-ray detector makes with a horizontal axis, said angle remaining constant in a single medical X-ray image (400);

b. selecting a plurality of pixels representing an object in said medical X-ray image (400);

c. determining magnification of each pixel in said medical X-ray image (400) using said stand geometry parameters, Angle$_{point}$, and an Angle$_{stand}$ values;

**characterized by**:

d. selectively reshaping said object in said medical X-ray image (400) to obtain a true shape of said object using said determined magnification,

wherein the instructions cause the server to perform the method steps comprising:

e. selecting a plurality of pixels representing a region of interest on said medical X-ray image (400);

f. selecting a reference pixel in said region of interest; and

g. adjusting said magnification of said plurality of pixels present in the surrounding of said reference pixel with respect to the magnification of said reference pixel.

8. The storage medium according to claim 7, wherein in selecting a plurality of pixels representing said object the instructions cause the server to perform the method steps comprising:

a. identifying a plurality of pixels representing said object in said medical X-ray image (400); and

b. selecting from said plurality of pixels a sequence of connecting pixels which represent a contour of said object.

9. The storage medium according to claim 8, wherein the instructions cause the server to further perform the method steps of determining said actual size of said object by calculating pixel spacing for each pixel representing said contour, wherein pixel spacing is the physical distance between the centers of two adjacent pixels, and wherein pixel spacing between adjacent pixels is determined using the magnification of the pixels.

**Patentansprüche**

1. Verfahren (200) zum Erhalten einer wahren Form eines Objekts in einem medizinischen Röntgenbild (400), wobei das Verfahren (200) umfasst:
Empfangen eines medizinischen Röntgenbildes (400) des Objekts;

   a. Empfangen von Röntgenstandgeometrie-Parameterwerten, einem Winkel$_{punkt}$ und einem Winkel$_{stand}$ Wert; wobei der Winkel-$_{punkt}$ ein Winkel ist, den eine Linie, die eine Röntgenquelle (401) mit einem gemessenen Punkt auf dem medizinischen Röntgenbild (400) verbindet, mit einer Linie bildet, die die Röntgenquelle (401) mit dem Zentrum des medizinischen Röntgenbildes (400) verbindet; und wobei der Winkel$_{stand}$ ein Winkel Z ist, den eine Linie, die die Röntgenquelle (401) und einen Röntgendetektor verbindet, mit einer horizontalen Achse bildet, wobei der Winkel in einem einzelnen medizinischen Röntgenbild (400) konstant bleibt;
   b. Auswählen einer Vielzahl von Pixeln, die das Objekt in dem medizinischen Röntgenbild darstellen (400);
   c. Bestimmen der Vergrößerung jedes Pixels in dem medizinischen Röntgenbild (400) unter Verwendung der Standgeometrieparameter, einem Winkel$_{punkt}$ und einem Winkel$_{stand}$ Wert;

   **gekennzeichnet durch**:

   d. Selektives Umformen des Objekts in dem medizinischen Röntgenbild (400), um eine echte Form des Objekts unter Verwendung der bestimmten Vergrößerung zu erhalten, wobei das Umformen des Objekts in dem medizinischen Röntgenbild (400) ferner umfasst:
   e. Auswählen einer Vielzahl von Pixeln, die eine Region von Interesse auf dem medizinischen Röntgenbild darstellen (400) ;
   f. Auswählen eines Referenzpixels in der Region von Interesse; und
   g. Anpassen der Vergrößerung der Vielzahl von Pixeln, die in der Umgebung des Referenzpixels vorhanden sind, in Bezug auf die Vergrößerung des Referenzpixels.

2. Verfahren (200) nach Anspruch 1, wobei das Verfahren bei der Auswahl einer Vielzahl von Pixeln, die das Objekt darstellen, umfasst:

   a. Identifizieren einer Vielzahl von Pixeln, die das Objekt in dem medizinischen Röntgenbild (400) darstellen; und
   b. Auswählen einer Sequenz von Verbindungspixeln, die eine Kontur des Objekts darstellen, aus der Vielzahl von Pixeln.

3. Verfahren (200) nach Anspruch 2, ferner umfassend ein Bestimmen einer tatsächlichen Größe des Objekts durch Berechnen eines Pixelabstands für jeden Pixel, der die Kontur darstellt, wobei der Pixelabstand der physikalische Abstand zwischen den Zentren zweier benachbarter Pixel ist, und
wobei der Pixelabstand zwischen benachbarten Pixeln unter Verwendung der Vergrößerung der Pixel bestimmt wird.

4. System (100) zum Erhalten einer wahren Form eines Objekts in einem medizinischen Röntgenbild (400), wobei das System (100) umfasst:

   a. eine Verarbeitungseinheit (101);
   b. eine Bilddatenbank (107), die mit der Verarbeitungseinheit (101) gekoppelt ist;
   c. einen Speicher (102), der mit der Verarbeitungseinheit (101) gekoppelt ist, wobei der Speicher (102) ein Vergrößerungskorrekturmodul (108) umfasst, das konfiguriert ist zum:

      Empfangen eines medizinischen Röntgenbildes (400) des Objekts;

      i. Empfangen von Röntgenstandgeometrie-Parameterwerten, einem Winkel$_{punkt}$ und einem Winkel$_{stand}$ Wert; wobei der Winkel$_{punkt}$ ein Winkel ist, den eine Linie, die eine Röntgenquelle (401) mit einem gemessenen Punkt auf dem medizinischen Röntgenbild (400) verbindet, mit einer Linie bildet, die die Röntgenquelle (401) mit dem Zentrum des medizinischen Röntgenbildes (400) verbindet; und wobei der Winkel$_{stand}$ ein Winkel Z ist, den eine Linie, die die Röntgenquelle (401) und einen Röntgendetektor verbindet, mit einer horizontalen Achse bildet, wobei der Winkel in einem einzelnen medizinischen Röntgenbild (400) konstant bleibt;
ii. Auswählen einer Vielzahl von Pixeln, die das Objekt in dem medizinischen Röntgenbild darstellen (400);
iii. Bestimmen der Vergrößerung jedes Pixels in dem medizinischen Röntgenbild (400) unter Verwendung der Standgeometrieparameter, einem $Winkel_{punkt}$ und einem $Winkel_{stand}$ Wert;

**dadurch gekennzeichnet, dass** das Vergrößerungskorrekturmodul (108) ferner konfiguriert ist zum:

Selektiven Umformen des Objekts in dem medizinischen Röntgenbild (400), um eine echte Form des Objekts unter Verwendung der bestimmten Vergrößerung zu erhalten,
wobei bei der selektiven Umformung des Objekts in dem medizinischen Röntgenbild (400) das Vergrößerungskorrekturmodul (108) konfiguriert ist zum:

    a. Auswählen einer Vielzahl von Pixeln, die eine Region von Interesse auf dem medizinischen Röntgenbild darstellen (400);
    b. Auswählen eines Referenzpixels in der Region von Interesse; und
    c. Anpassen der Vergrößerung der Vielzahl von Pixeln, die in der Umgebung des Referenzpixels vorhanden sind, in Bezug auf die Vergrößerung des Referenzpixels.

5.  System (100) nach Anspruch 4, wobei bei der Auswahl einer Vielzahl von Pixeln, die das Objekt darstellen, das Vergrößerungskorrekturmodul (108) konfiguriert ist zum:

    a. Identifizieren einer Vielzahl von Pixeln, die das Objekt in dem medizinischen Röntgenbild (400) darstellen; und
    b. Auswählen einer Sequenz von Verbindungspixeln, die eine Kontur des Objekts darstellen, aus der Vielzahl von Pixeln.

6.  System (100) nach Anspruch 5, wobei das Vergrößerungskorrekturmodul ferner konfiguriert zum Bestimmen einer tatsächlichen Größe des Objekts durch Berechnen eines Pixelabstands für jeden Pixel, der die Kontur darstellt, wobei der Pixelabstand der physikalische Abstand zwischen den Zentren zweier benachbarter Pixel ist, und wobei der Pixelabstand zwischen benachbarten Pixeln unter Verwendung der Vergrößerung der Pixel bestimmt wird.

7.  Ein nicht transitorisches computerlesbares Speichermedium mit darin gespeicherten maschinenlesbaren Anweisungen, die, wenn sie von einem Server ausgeführt werden, den Server veranlassen, die Verfahrensschritte auszuführen, die folgendes umfassen:
Empfangen eines medizinischen Röntgenbildes (400) eines Objekts;

    a. Empfangen von Röntgenstandgeometrie-Parameterwerten, einem $Winkel_{punkt}$ und einem $Winkel_{stand}$ Wert; wobei der $Winkel_{punkt}$ ein Winkel ist, den eine Linie, die eine Röntgenquelle (401) mit einem gemessenen Punkt auf dem medizinischen Röntgenbild (400) verbindet, mit einer Linie bildet, die die Röntgenquelle (401) mit dem Zentrum des medizinischen Röntgenbildes (400) verbindet; und wobei der $Winkel_{stand}$ ein Winkel Z ist, den eine Linie, die die Röntgenquelle (401) und einen Röntgendetektor verbindet, mit einer horizontalen Achse bildet, wobei der Winkel in einem einzelnen medizinischen Röntgenbild (400) konstant bleibt;
    b. Auswählen einer Vielzahl von Pixeln, die das Objekt in dem medizinischen Röntgenbild darstellen (400);
    c. Bestimmen der Vergrößerung jedes Pixels in dem medizinischen Röntgenbild (400) unter Verwendung der Standgeometrieparameter, einem $Winkel_{punkt}$ und einem $Winkel_{stand}$ Wert; **gekennzeichnet durch**:
    d. Selektives Umformen des Objekts in dem medizinischen Röntgenbild (400), um eine echte Form des Objekts unter Verwendung der bestimmten Vergrößerung zu erhalten, wobei die Anweisungen den Server veranlassen, die Verfahrensschritte auszuführen, umfassend:

    e. Auswählen einer Vielzahl von Pixeln, die eine Region von Interesse auf dem medizinischen Röntgenbild darstellen (400) ;
    f. Auswählen eines Referenzpixels in der Region von Interesse; und
    g. Anpassen der Vergrößerung der Vielzahl von Pixeln, die in der Umgebung des Referenzpixels vorhanden sind, in Bezug auf die Vergrößerung des Referenzpixels.

8.  Speichermedium nach Anspruch 7, wobei bei der Auswahl einer Vielzahl von Pixeln, die das Objekt darstellen, die Anweisungen den Server veranlassen, die Verfahrensschritte auszuführen, umfassend:

a. Identifizieren einer Vielzahl von Pixeln, die das Objekt in dem medizinischen Röntgenbild (400) darstellen; und

b. Auswählen einer Sequenz von Verbindungspixeln, die eine Kontur des Objekts darstellen, aus der Vielzahl von Pixeln.

9. Speichermedium nach Anspruch 8, wobei die Anweisungen den Server veranlassen, ferner die Verfahrensschritte auszuführen zur Bestimmung einer tatsächlichen Größe des Objekts durch Berechnen eines Pixelabstands für jeden Pixel, der die Kontur darstellt, wobei der Pixelabstand der physikalische Abstand zwischen den Zentren zweier benachbarter Pixel ist, und wobei der Pixelabstand zwischen benachbarten Pixeln unter Verwendung der Vergrößerung der Pixel bestimmt wird.

**Revendications**

1. Procédé (200) pour obtenir une forme réelle d'un objet dans une image radiographique médicale (400), le procédé (200) comprenant :
   recevoir une image radiographique médicale (400) de l'objet ;

   a. recevoir des valeurs de paramètres de géométrie du statif de rayons X, un $angle_{point}$ et une valeur $angle_{statif}$ ; dans lequel ledit $angle_{point}$ est un angle qu'une ligne reliant une source de rayons X (401) à un point mesuré sur ladite image radiographique médicale (400) fait avec une ligne reliant la source de rayons X (401) au centre de ladite image radiographique médicale (400) ; et dans lequel $angle_{statif}$ est un angle Z qu'une ligne reliant ladite source de rayons X (401) et un détecteur de rayons X fait avec un axe horizontal, ledit angle restant constant dans une seule image radiographique médicale (400) ;
   b. sélectionner une pluralité de pixels représentant ledit objet dans ladite image radiographique médicale (400) ;
   c. déterminer le grossissement de chaque pixel dans ladite image radiographique médicale (400) en utilisant lesdits paramètres de géométrie du statif, $angle_{point}$, et une valeur $angle_{statif}$ ;

   **caractérisé par** :

   d. remodeler sélectif ledit objet dans ladite image radiographique médicale (400) pour obtenir une forme réelle dudit objet en utilisant ledit grossissement déterminé, dans lequel le remodelage dudit objet dans ladite image radiographique médicale (400) comprend en outre :
   e. sélectionner une pluralité de pixels représentant une région d'intérêt sur ladite image radiographique médicale (400) ;
   f. sélectionner un pixel de référence dans ladite région d'intérêt ; et
   g. ajuster ledit grossissement de ladite pluralité de pixels présents dans l'environnement dudit pixel de référence par rapport au grossissement dudit pixel de référence.

2. Procédé (200) selon la revendication 1, dans lequel, en sélectionnant une pluralité de pixels représentant ledit objet, ledit procédé comprend :

   a. identifier une pluralité de pixels représentant ledit objet dans ladite image radiographique médicale (400) ; et
   b. sélectionner parmi ladite pluralité de pixels une séquence de pixels de connexion qui représente un contour dudit objet.

3. Procédé (200) selon la revendication 2, comprenant en outre la détermination d'une taille réelle dudit objet en calculant l'espacement des pixels pour chaque pixel représentant ledit contour, dans lequel l'espacement des pixels est la distance physique entre les centres de deux pixels adjacents, et
   où l'espacement des pixels entre des pixels adjacents est déterminé par le grossissement des pixels.

4. Un système (100) pour obtenir une forme réelle d'un objet dans une image radiographique médicale (400), ledit système (100) comprenant :

   a. une unité de traitement (101) ;
   b. une base de données d'images (107) couplée à ladite unité de traitement (101) ;
   c. une mémoire (102) couplée à ladite unité de traitement (101), ladite mémoire (102) comprenant un module de correction de grossissement (108) configuré pour :

recevoir une image radiographique médicale (400) de l'objet ;

i. recevoir des valeurs de paramètres de géométrie du statif de rayons X, un $angle_{point}$ et une valeur $angle_{statif}$ ; dans lequel ledit $angle_{point}$ est un angle qu'une ligne reliant une source de rayons X (401) à un point mesuré sur ladite image radiographique médicale (400) fait avec une ligne reliant la source de rayons X (401) au centre de ladite image radiographique médicale (400) ; et dans lequel $angle_{statif}$ est un angle Z qu'une ligne reliant ladite source de rayons X (401) et un détecteur de rayons X fait avec un axe horizontal, ledit angle restant constant dans une seule image radiographique médicale (400) ;

ii. sélectionner une pluralité de pixels représentant ledit objet dans ladite image radiographique médicale (400) ;

iii. déterminer le grossissement de chaque pixel dans ladite image radiographique médicale (400) en utilisant lesdits paramètres de géométrie du statif, $angle_{point}$, et une valeur $angle_{statif}$ ;

**caractérisé en ce que** ledit module de correction de grossissement (108) est en outre configuré pour

remodeler sélectif ledit objet dans ladite image radiographique médicale (400) pour obtenir une forme réelle dudit objet en utilisant ledit grossissement déterminé,

dans lequel en remodelant sélectivement ledit objet dans ladite image radiographique médicale (400), ledit module de correction de grossissement (108) est configuré pour :

a. sélectionner une pluralité de pixels représentant une région d'intérêt sur ladite image radiographique médicale (400);

b. sélectionner un pixel de référence dans ladite région d'intérêt ; et

c. ajuster ledit grossissement de ladite pluralité de pixels présents dans l'environnement dudit pixel de référence par rapport au grossissement dudit pixel de référence.

5. Le système (100) selon la revendication 4, dans lequel, en sélectionnant une pluralité de pixels représentant ledit objet, ledit module de correction de grossissement (108) est configuré pour :

a. identifier une pluralité de pixels représentant ledit objet dans ladite image radiographique médicale (400) ; et

b. sélectionner parmi ladite pluralité de pixels une séquence de pixels de connexion qui représente un contour dudit objet.

6. Système (100) selon la revendication 5, dans lequel ledit module de correction de grossissement est en outre configuré pour déterminer une taille réelle dudit objet en calculant l'espacement des pixels pour chaque pixel représentant ledit contour, dans lequel l'espacement des pixels est la distance physique entre les centres de deux pixels adjacents, et

où l'espacement des pixels entre des pixels adjacents est déterminé par le grossissement des pixels.

7. Un support de stockage non transitoire lisible par ordinateur sur lequel sont stockées des instructions lisibles par machine qui, lorsqu'elles sont exécutées par un serveur, font que le serveur exécute les étapes du procédé comprenant :

recevoir une image radiographique médicale (400) d'un objet ;

a. recevoir des valeurs de paramètres de géométrie du statif de rayons X, un $angle_{point}$ et une valeur $angle_{statif}$ ; dans lequel ledit $angle_{point}$ est un angle qu'une ligne reliant une source de rayons X (401) à un point mesuré sur ladite image radiographique médicale (400) fait avec une ligne reliant la source de rayons X (401) au centre de ladite image radiographique médicale (400) ; et dans lequel $angle_{statif}$ est un angle Z qu'une ligne reliant ladite source de rayons X (401) et un détecteur de rayons X fait avec un axe horizontal, ledit angle restant constant dans une seule image radiographique médicale (400) ;

b. sélectionner une pluralité de pixels représentant ledit objet dans ladite image radiographique médicale (400) ;

c. déterminer le grossissement de chaque pixel dans ladite image radiographique médicale (400) en utilisant lesdits paramètres de géométrie du statif, $angle_{point}$, et une valeur $angle_{statif}$ ;

**caractérisé par** :

d. remodeler sélectif ledit objet dans ladite image radiographique médicale (400) pour obtenir une forme réelle dudit objet en utilisant ledit grossissement déterminé,

EP 3 457 353 B1

où les instructions font que le serveur exécute les étapes du procédé comprenant :

> e. sélectionner une pluralité de pixels représentant une région d'intérêt sur ladite image radiographique médicale (400) ;
> f. sélectionner un pixel de référence dans ladite région d'intérêt ; et
> g. ajuster ledit grossissement de ladite pluralité de pixels présents dans l'environnement dudit pixel de référence par rapport au grossissement dudit pixel de référence.

**8.** Support de stockage selon la revendication 7, dans lequel, en sélectionnant une pluralité de pixels représentant ledit objet, les instructions font que le serveur exécute les étapes du procédé comprenant :

> a. identifier une pluralité de pixels représentant ledit objet dans ladite image radiographique médicale (400) ; et
> b. sélectionner parmi ladite pluralité de pixels une séquence de pixels de connexion qui représente un contour dudit objet.

**9.** Support de stockage selon la revendication 8, dans lequel les instructions font que le serveur exécute en outre les étapes du procédé comprenant la détermination d'une taille réelle dudit objet en calculant l'espacement des pixels pour chaque pixel représentant ledit contour, dans lequel l'espacement des pixels est la distance physique entre les centres de deux pixels adjacents, et

où l'espacement des pixels entre des pixels adjacents est déterminé par le grossissement des pixels.

# FIG 1

100

```
┌─────────────────────────────────────────────────────────┐
│                                                           │
│   ┌─────────┐          ┌──────┐         ┌───────────┐    │
│   │   101   │◄────────►│      │◄───────►│    102    │    │
│   └─────────┘          │      │         │           │    │
│                        │      │         │  ┌─────┐  │    │
│                        │      │         │  │ 108 │  │    │
│   ┌─────────┐          │      │         │  └─────┘  │    │
│   │   103   │          │ 105  │         └───────────┘    │
│   │         │          │      │                          │
│   │ ┌─────┐ │◄────────►│      │◄───────► ┌──────┐        │
│   │ │ 107 │ │          │      │          │ 104  │        │
│   │ └─────┘ │          │      │          └──────┘        │
│   └─────────┘          │      │                          │
│                        │      │◄───────► ┌──────┐        │
│                        │      │          │ 106  │        │
│                        └──────┘          └──────┘        │
│                                                           │
└─────────────────────────────────────────────────────────┘
```

# FIG 2

200

```
        ┌──────────────────┐
        │       201        │
        └──────────────────┘
                 │
                 ▼
        ┌──────────────────┐
        │       202        │
        └──────────────────┘
                 │
                 ▼
        ┌──────────────────┐
        │       203        │
        └──────────────────┘
                 │
                 ▼
        ┌──────────────────┐
        │       204        │
        └──────────────────┘
                 │
                 ▼
        ┌──────────────────┐
        │       205        │
        └──────────────────┘
```

# FIG 3

300

301

302

303

304

# FIG 4A

400

# FIG 4B

| Measurement # | Value | Error % |
|---|---|---|
| X1 | 31.05 | 2.66 |
| X2 | 27.29 | 14.45 |
| X3 | 24.35 | 23.67 |

FIG 4C

# FIG 5

500

ERROR IN MEASURED VALUE (%)

| | ROI Left | ROI Center | ROI Right | Proposed Method |

Bar chart values:
- ROI Left: -1.38 %, -13.71 %, -24.09 %
- ROI Center: 13.64 %, -0.56 %, -12.52 %
- ROI Right: 29.77 %, 13.55 %, -0.13 %
- Proposed Method: -0.72 %, 0.19 %, 0.72 %

Legend: Error Left Sphere, Error Center Sphere, Error Right Sphere

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008063304 A1

**Non-patent literature cited in the description**

- **J. T. DOBBINS III ; D. J. GODFREY.** Digital x-ray tomosynthesis: current state of the art and clinical potential. *Phys. Med. Biol.,* 2003, vol. 48

- **Z. KOLITSI et al.** A multiple projection method for digital tomosynthesis. *Med. Phys.,* 1992, vol. 19, 4